(19) Europäisches Patentamt
European Patent Office
Office européen des brevets

(11) Veröffentlichungsnummer: **0 166 875**
**A1**

(12) # EUROPÄISCHE PATENTANMELDUNG

(21) Anmeldenummer: **85103955.2**

(22) Anmeldetag: **02.04.85**

(51) Int. Cl.[4]: **C 07 D 403/12**
A 61 K 31/495

(30) Priorität: **06.04.84 CS 2662/84**

(43) Veröffentlichungstag der Anmeldung:
**08.01.86 Patentblatt 86/2**

(84) Benannte Vertragsstaaten:
**AT BE CH DE FR GB LI NL SE**

(71) Anmelder: **SPOFA Spojené Podniky Pro Zdravotnickou Vyrobu**
**Husinecká 11 a**
**Prag 3(CS)**

(72) Erfinder: **Fisnerová, Ludmila, Dipl.-Ing. CSc**
**Zvonková 2640**
**Praha 10(CS)**

(72) Erfinder: **Grimova, Jaroslava, MUDr, CSc**
**Na Doubkové 2**
**Praha 5(CS)**

(72) Erfinder: **Roubal, Zdenek, RNDr, CSc**
**Ruzová 7**
**Praha 1(CS)**

(72) Erfinder: **Nemecek, Oldrich, Dipl.-Ing., DrSc**
**Novorosijská 7**
**Praha 10(CS)**

(74) Vertreter: **Patentanwälte Beetz sen. - Beetz jun. Timpe - Siegfried - Schmitt-Fumian**
**Steinsdorfstrasse 10**
**D-8000 München 22(DE)**

(54) **3-2-(2-Benzimidazolyl)-benzoyloxyethyl-4(3H)-chinazolinon, Verfahren zu seiner Herstellung und diese Verbindung enthaltende Arzneipräparate.**

(57) Die Erfindung betrifft 3-[2-(2-Benzimidazolyl)-benzoyloxyethyl]-4(3H)-chinazolinon der Formel I. Diese Verbindung wird durch Acylierung von 3-(Hydroxyethyl)-4(3H)-chinazolinon mit einem reaktionsfähigen Derivat der 2-(2-Benzimidazolyl)-benzoesäure und vorzugsweise mit ihrem Imidazolid hergestellt. Dazu wird die 2-(2-Benzimidazolyl)-benzoesäure zuerst mit 1,1'-Carbonyldiimidazol umgesetzt, dann wird das gebildete Imidazolid in situ mit 3-(2-Hydroxyethyl)-4(3H)-chinazolinon umgesetzt. Die Isolierung und Reinigung des Reaktionsprodukts erfolgt in an sich bekannter Weise.

Die Verbindung der Formel I weist bemerkenswerte analgetische Eigenschaften in Kombination mit einer äußerst geringen Toxizität auf und ist daher als Analgetikum für Arzneipräparate geeignet.

EP 0 166 875 A1

Croydon Printing Company Ltd

# 3-[2-(2-Benzimidazolyl)-benzoyloxyethyl]-4(3H)-chinazolinon, Verfahren zu seiner Herstellung und diese Verbindung enthaltende Arzneipräparate

Die Erfindung betrifft 3-[2-(2-Benzimidazolyl)-benzoyloxyethyl]-4(3H)-chinazolinon der Formel I

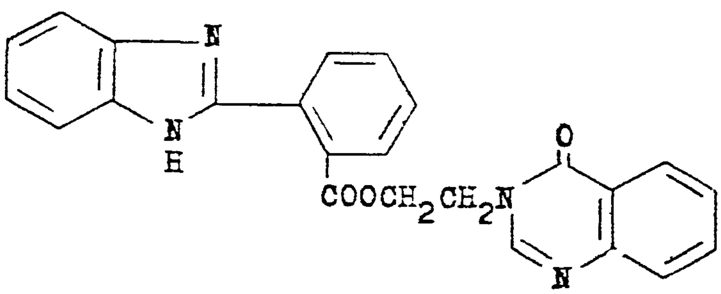

(I).

Bei pharmakologischen Vergleichsversuchen mit Aminophenazon (2,3-Dimethyl-4-dimethylamino-1-phenyl-5-pyrazolon) wies die erfindungsgemäße Verbindung der Formel 1 überraschenderweise eine ausgeprägte analgetische Aktivität und eine wesentlich geringere Toxizität auf. Bei der Bestimmung der analgetischen Aktivität an Mäusen nach der bekannten Testmethode hatte die erfindungsgemäße Verbindung der Formel I nach oraler Verabreichung einen $ED_{50}$-Wert von 115 mg/kg, Aminophenazon dagegen einen $ED_{50}$-Wert von 104 mg/kg. Die erfindungsgemäße Verbindung der Formel I weist bei der oralen Verabreichung an Ratten in Einzeldosen von 100 bzw 200 mg/kg eine statistisch signifikante entzündungshemmende Wirkung auf. Die Verbindung der Formel I ist praktisch nicht toxisch: ihr $LD_{50}$-Wert liegt außerhalb des gewöhnlichen Experimentalbereichs ($LD_{50} > 8$ g/kg bei oraler Verabreichung an Mäuse ).

Durch ihre stark ausgeprägte analgetische Aktivität in Kombination mit der äußerst geringen Toxizität ist die erfindungsgemäße Verbindung der Formel I als Analgetikum geeignet.

Die Erfindung betrifft daher auch Arzneipräparate, die durch eine Verbindung der Formel I als Wirkstoff gekennzeichnet sind.

Die Verbindung der Formel I wird erfindungsgemäß durch Acylierung von 3-(2-Hydroxyethyl)-4(3H)-chinazolinon der Formel II

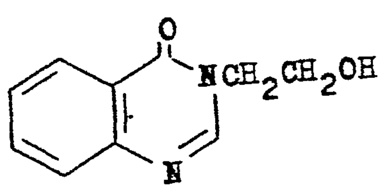

(II)

mit einem reaktionsfähigen Derivat der 2-(2-Benzimidazolyl)-benzoesäure der Formel III und vorzugsweise mit deren Imidazolid hergestellt

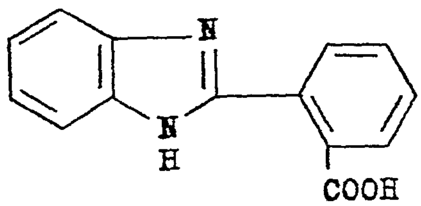

(III).

Dazu wird die 2-(2-Benzimidazolyl)-benzoesäure in an sich bekannter Weise mit 1,1'-Carbonyldiimidazol umgesetzt; das gebildete Imidazolid wird dann in situ mit 3-(2-Hydroxyethyl)-4(3H)-chinazolinon umgesetzt und zwar zweckmäßigerweise in einem inerten organischen Lösungsmittel, vorzugsweise in einem chlorierten $C_{1-2}$-Alkan, wie Dichlormethan, und im wesentlichen bei Raumtemperatur. Das dabei freigesetzte Imidazol wird aus dem Reaktionsgemisch in an sich bekannter Weise mit Wasser ausgewaschen. Durch Abdampfen des Lösungsmittels wird das gewünschte Produkt erhalten, das nötigenfalls durch Kristallisation gereinigt wird.

Die Erfindung wird durch das Beispiel erläutert. (Die Ausgangsverbindungen stellen bekannte Verbindungen dar, die in der einschlägigen Literatur bereits beschrieben worden sind).

<u>Ausführungsbeispiel</u>

17,1 g 1,1'-Carbonyldiimidazol werden bei 20 bis 30 °C in 300 ml wasserfreiem Dichlormethan gelöst und mit 25,2 g 2-(2-Benzimidazolyl)-benzoesäure versetzt. Das Gemisch wird bis zur vollständigen Lösung gerührt, mit 20 g 3-(2-Hydroxyethyl)-4(3H)-chinazolinon

versetzt, bei 20 bis 30 °C 6 h gerührt, über Nacht stehengelassen und dann noch weitere 8 h gerührt. Die abgekühlte Lösung wird mit Wasser gewaschen und unter vermindertem Druck zur Trockne eingedampft. Der Rückstand wird aus 2-Propanol umkristallisiert. Ausbeute: 30 g (70 % d.Th.), Fp. 192 bis 193 °C.

## Ansprüche

1. 3-[2-(2-Benzimidazolyl)-benzoyloxyethyl]-4(3H)-chinazolinon der Formel I

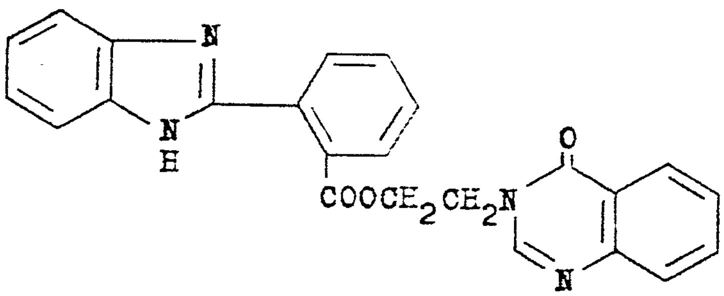

(I).

2. Verfahren zur Herstellung von 3-[2-(2-Benzimidazolyl)-benzoyloxyethyl]-4(3H)-chinazolinon der Formel I gemäß Anspruch 1,

dadurch gekennzeichnet,

daß 3-(2-Hydroxyethyl)-4(3H)-chinazolinon der Formel II

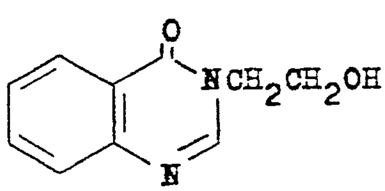

(II)

mit einem reaktionsfähigen Derivat der 2-(2-Benzimidazolyl)-benzoesäure der Formel III acyliert wird

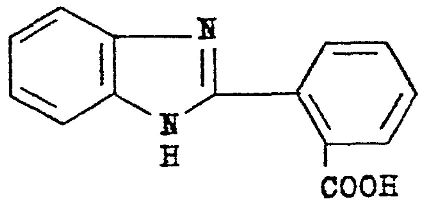 .

3. Verfahren nach Anspruch 2, dadurch gekennzeichnet, daß zuerst 2-(2-Benzimidazolyl)-benzoesäure in an sich bekannter Weise mit 1,1'-Carbonyldiimidazol umgesetzt, das gebildete Imidazolid mit 3-(2-Hydroxyethyl)-4(3H)-chinazolinon umgesetzt, das dabei freigesetzte Imidazol aus dem Reaktionsgemisch ausgewaschen und 3-[2-(2-Benzimidazolyl)-benzoyloxyethyl]-4(3H)-chinazolinon durch Einengen isoliert wird.

4. Verfahren nach Anspruch 2 oder 3, dadurch gekennzeichnet, daß die Umsetzung in einem chlorierten $C_{1-2}$-Alkan bei Raumtemperatur durchgeführt wird.

5. Arzneipräparat, gekennzeichnet durch eine Verbindung der Formel I gemäß Anspruch 1 als Wirkstoff.

Ansprüche

Für Österreich

1. Verfahren zur Herstellung von 3-[2-(2-Benzimidazolyl)-benzoyloxyethyl]-4(3H)-chinazolinon der Formel I

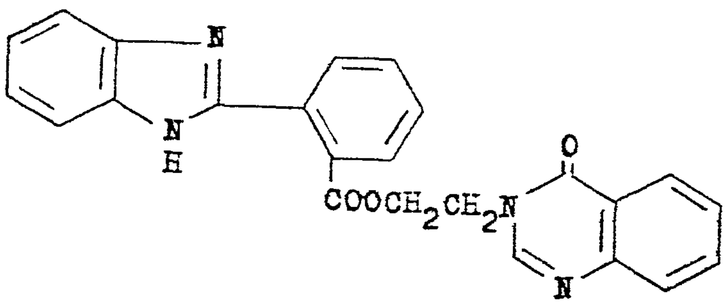

(I),

dadurch gekennzeichnet,

daß 3-(2-Hydroxyethyl)-4(3H)-chinazolinon der Formel II

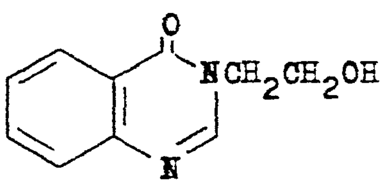

(II)

mit einem reaktionsfähigen Derivat der 2-(2-Benzimidazolyl)-benzoesäure der Formel III acyliert wird

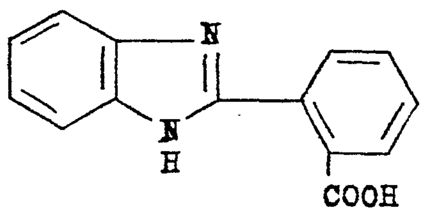

(III).

2. Verfahren nach Anspruch 1, dadurch gekennzeichnet, daß zuerst 2-(2-Benzimidazolyl)-benzoesäure in an sich bekannter Weise mit 1,1'-Carbonyldiimidazol umgesetzt, das gebildete Imidazolid mit 3-(2-Hydroxyethyl)-4(3H)-chinazolinon umgesetzt, das dabei freigesetzte Imidazol aus dem Reaktionsgemisch ausgewaschen und 3-[2-(2-Benzimidazolyl)-benzoyloxyethyl]-4(3H)-chinazolinon durch Einengen isoliert wird.

3. Verfahren nach Anspruch 1 oder 2, dadurch gekennzeichnet, daß die Umsetzung in einem chlorierten $C_{1-2}$-Alkan bei Raumtemperatur durchgeführt wird.

4. Arzneipräparat, gekennzeichnet durch eine Verbindung der Formel I, hergestellt gemäß einem der Ansprüche 1 bis 3, als Wirkstoff.

Europäisches Patentamt

# EUROPÄISCHER RECHERCHENBERICHT

0166875
Nummer der Anmeldung
EP 85 10 3955

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| | Keine Entgegenhaltungen<br>----- | | C 07 D 403/12<br>A 61 K 31/495 |
| | | | RECHERCHIERTE SACHGEBIETE (Int. Cl.4) |
| | | | C 07 D 403/00<br>A 61 K 31/00 |

Der vorliegende Recherchenbericht wurde für alle Patentansprüche erstellt

| Recherchenort | Abschlußdatum der Recherche | Prüfer |
|---|---|---|
| DEN HAAG | 01-07-1985 | DE BUYSER I.A.F. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03.82